# EUROPEAN PATENT SPECIFICATION

(11) **EP 2 618 768 B1**
(45) Date of publication and mention of the grant of the patent: **14.01.2015**
(21) Application number: 10759591.0
(22) Date of filing: 24.09.2010
(51) Int. Cl.: A61B 18/20, A61N 5/06, A61B 18/22, A61N 5/067

(54) **LASER SYSTEM FOR TREATMENT OF BODY TISSUE**
LASERSYSTEM ZUR BEHANDLUNG VON KÖRPERGEWEBE
SYSTÈME LASER POUR LE TRAITEMENT DE TISSU CORPOREL

(43) Date of publication of application: 31.07.2013
(73) Proprietor: Fotona d.d., 1210 Ljubljana (SI)
(72) Inventor: LUKAC, Matjaz, SI-1000 Ljubljana (SI); KAZIC, Marko, SI-1233 Dob (SI)
(74) Representative: Zurhorst, Stefan
(86) International application number: PCT/EP2010/005853
(87) International publication number: WO 2012/037954

(56) References cited:
- EP-A1- 0 673 627
- EP-A2- 0 821 916
- WO-A1-2008/111970
- GB-A- 1 537 696
- US-A1- 2004 078 032
- US-A1- 2007 191 682
- US-A1- 2009 209 824
- US-B1- 6 501 551
- YEOW, YANG, CHAHWAN, GORDON, QI, VITKIN, WILSON, GOLDBERG: "Micromachined 2-D scanner for 3-D optical coherence tomography", SENSORS AND ACTUATORS A, vol. 117, 29 July 2004 (2004-07-29), pages 331-340, XP002612284,

## Description

The invention relates to a device for the treatment of body tissue on an inner circumferential body surface.

Various laser wavelengths, such as Er:YAG (2.94 µm wavelength), Er,Cr:YSGG (2.73 µm wavelength), CO₂ (8-11 µm wavelength) have been advocated as possible and promising alternatives to conventional instruments in different medical and surgical disciplines. Due to their characteristic absorption maxima and thermal absorption coefficients, laser systems are considered to be suitable not only for the treatment of soft tissue structures but also for mineralized hard tissues. Contact-free laser ablation offers the opportunity of cutting bone tissue and other hard tissue without friction that may cause an additional thermal and mechanical trauma. Consequently, the risk of cell death and delayed healing may be minimized. Furthermore, in contrast to conventional procedures no tissue particles debris is left on the tissue surface leading to a smear layer on the treated surface. This is due to the laser tissue ablation mechanism. Absorption and the following transformation of laser irradiation into heat results in a rapid phase change which, in turn, creates internal pressures, causing micro-fracturing and micro-explosive removal of the mineral phase of the hard tissues. Besides, during the laser ablation of the tissue, the vaporization of water leads to a fast removal of the tissue layers. The result is an extremely clean and micro-structured tissue surface without thermal damage and smear layer, resulting in the reduced inflammatory response and accelerated tissue regeneration and attachment. This is for example important in implantology where faster attachment of the bone to the inserted implants is crucial for faster patient recovery time.

Nevertheless, drawbacks of laser hard tissue surgery such as a considerable amount of time needed, a missing depth control and highly sophisticated handling requirements are still considerable. An advantage of mechanical tools such as drills and saws is that the surgeon has a very good tactile contact with the treated tissue providing feedback to the surgeon regarding the speed of the procedure and the depth of the drilled hole or cut. For this reason, laser bone cutting is still assessed to be inferior to many conventional as well as other methods, such as piezoelectric osteotomy.

In particular, when creating a hole in hard body tissue like bone material, mechanical tools are commonly still preferred. This preference however leaves open the handling of the a.m. issues like e.g. residual smear layers on the inner circumferential body tissue surface of said hole. The treatment results of the inner circumferential body tissue surface by mechanical means are unsatisfactory.

Similarly, it is sometimes desirable to use laser light to treat soft or hard tissues on difficult to reach inner circumferential body surfaces. Treatments may involve skin ablation, incision, excision, vaporization, coagulation, tightening, hemostasis or disinfection, and can be performed for example in vaginal, urinal, rectal, ENT (ear, nose and throat) and other procedures. The choice of the laser wavelength depends on a procedure and the desired effect on the tissue, and is not limited to the hard tissue wavelengths mentioned above. For example, when hemostasis is desired, the surgeon may decide to use Nd:YAG (1.064 µm wavelength) or KTP:YAG (0.532 µm wavelength) lasers.

EP 0 821 916 A2 discloses a laser system comprising a treatment head extending along a longitudinal axis. During operation, the laser beam enters the treatment head in the direction of the longitudinal axis. A planar deflection mirror is disposed in the treatment head at an angle of 45° to the longitudinal axis and guides the laser beam radially outwards out of the treatment head. In order to treat arcs on a cylindrical surface, a concerted movement of the deflection mirror and a beam focusing lens is required.

Similarly, EP 0 673 627 A1 discloses an optical fibre catheter for directing a laser beam to a treatment area. The distal fibre tip mirrors the incoming laser beam into a radial direction onto the treatment area. The fibre including its mirror tip may be turned about the longitudinal axis for a circumferential laser appliance.

The invention has the object to provide means for treating body tissue on an inner circumferential body tissue surface, which are able to improve or modify the surface tissue, thereby giving tactile contact and feedback to the surgeon.

This object is solved by a laser system with the features of claim 1.

A laser system for treatment of body tissue on an inner circumferential tissue surface comprises a laser source for generation of a laser beam, and a handpiece with a treatment head. The treatment head extends along a longitudinal axis. The treatment head is adapted in a manner, that the longitudinal axis of the treatment head during operation is at least approximately parallel to the inner circumferential tissue surface. During operation, the laser beam enters the treatment head in the direction of the longitudinal axis. A deflection mirror is disposed in the treatment head and guides the laser beam radially outwards out of the treatment head onto the inner circumferential tissue surface. A movable deflection means for the laser beam is provided, wherein the movable deflection means comprises a scanner being movable about two axes. The deflection mirror has a conical shape and is disposed with its apex facing the scanner. The scanner is controlled for at least a circular scanning of the conical deflection mirror, thereby scanning the inner circumferential tissue surface within a treatment area at least in a circumferential direction.

The inventive device allows for an access to tissue surfaces, which are not easily accessible, e.g. in the inner side of a hole or a body cavity. The slim treatment head of the handpiece may be axially inserted into the hole or into the body cavity, thereby contacting the inner circumferential tissue surface. Despite of the cramped spatial conditions, the deflection mirror provides, supported by the contacting of the treatment head with the inner circumferential tissue surface, that the focus of the laser beam is projected with a high fluence on the inner circumferential tissue surface, thereby providing the desired tissue treatment. By means of the movable deflection means, a predetermined scanning pattern may be scanned, which leads to the desired treatment result.

The inventive laser system is in particular suitable for the post treatment of drilling holes in hard bone material e. g. in implantology. At first, the drilling hole is mechanically produced, as usual. Subsequently, the laser system is used as a laser grater or laser rasp, by means of which residual smear layers of loose bone material are cleared from the inner circumferential tissue surface of the drilling hole. In addition, the drilling hole may be brought to its nominal measure with an improved precision. Finally, it is possible to achieve a desired surface quality and even a desired surface structuring. In addition to the treatment of hard bone material, the inventive laser system is suitable for the treatment of soft body tissue like skin or the like, in particular for vaginal, urinal, rectal or ENT (ear, nose and throat) treatments.

The deflection means comprises a scanner being movable about two axes, wherein the deflection mirror has a conical shape and is disposed with its apex facing the scanner.

According to this aspect of the invention, a mechanically rotated deflection mirror or a mechanically rotated treatment head is not required. By means of the scanner which is movable about two axes, the laser beam is guided over the conical reflection surface of the conical deflection mirror in such a manner, that the inner circumferential tissue surface is scanned both in the circumferential direction and in the axial direction, thereby following a certain scanning pattern without limitations. Within the treatment head, no mechanically movable parts are present. The treatment head itself does not perform any mechanically driven movement either; as a consequence thereof, the inner circumferential tissue surface does not have any contact to mechanically movable parts. The treatment head may be designed slim with low construction volume. This allows treatment of even very small drill holes or openings. By means of a suitable scanner control device for the two axial scanner, even complex scanning patterns may be achieved. Such scanning patterns do not necessarily need to be evenly distributed in the circumferential direction. Referring to the circumferential direction, certain angular sections may be excluded from the laser treatment.

It can be expedient that the laser system comprises a carrier, and that the deflection mirror is fixed to the treatment head by means of the carrier, wherein the carrier comprises carrier arms and windows between the carrier arms for the emerging laser beam.

This provides a mechanically simple means for secure fixing of the deflection mirror to the treatment head. The laser beam exits from the treatment head through said window onto the treatment area without any optical disturbance. The carrier arms may be designed sufficiently thin; accordingly their shadowing effect is negligible.

In the alternative, it may be preferred that the laser system comprises a carrier, and that the deflection mirror is fixed to the treatment head by means of the carrier, wherein the carrier is transparent and closed in the circumferential direction.

The transparent carrier may be manufactured of an optical glass or other suitable material being transparent for the laser beam. The laser beam may exit the treatment head in any desired angle referred to the circumferential direction, without any shadowing effect. In addition to carrying the deflection mirror, the transparent carrier acts as a protection device for the deflection mirror and the inner space of the treatment head, thereby preventing any pollution by body tissue debris.

In a preferred embodiment, the movable deflection means comprises a control device adapted to scan the treatment area by the laser beam on circles with centres on the treatment area, wherein the laser beam is subjected to a circular feed about the longitudinal axis of the treatment head.

Alternatively, it may be expedient that the deflection means comprises a control device adapted to scan the treatment area by the laser beam parallel to the longitudinal axis of the treatment head, wherein the laser beam is subjected to a circular feed about the longitudinal axis of the treatment head.

In a further alternative, it may be preferable that the deflection means comprises a control device adapted to scan the treatment area by the laser beam in a random pattern, wherein the laser beam is subjected to a circular feed about the longitudinal axis of the treatment head.

A random pattern, combined with a circular feed about the longitudinal axis of the treatment head, results in a more homogenous treatment of the circumferential tissue surface. The homogeneity may be further improved by slight longitudinal and/or rotational movements of the handpiece.

In a still further alternative, it may be preferable that the deflection means comprises a control device adapted to scan the treatment area by the laser beam solely in a circular way about the longitudinal axis of the treatment head.

The latter alternative leads to reduction of a circular scanning pattern being scanned in a circular way about the longitudinal axis of the treatment head. As a consequence, the control effort for the scanner can be minimized. In connection with a rotating deflection mirror, an additional scanner may be entirely omitted. By providing a laser beam profile with a sufficient large diameter and preferably with a top hat shaped beam profile, the treatment area is evenly and homogenously illuminated, without need for manual corrections.

Embodiments of the invention will be explained in the following with the aid of the drawings in more detail. It is shown in:
- Fig. 1: in a schematic sectional view, a laser system with a rotationally drivable treatment head carrying a deflection mirror;
- Fig. 2: in a schematic sectional view, an embodiment of an inventive laser system with deflection means comprising a scanner being movable about two axes and a deflection mirror having a conical shape, with the deflection mirror being fixed to the treatment head by a transparent carrier;
- Fig. 3: a variant of the system according to Fig. 2 as a third embodiment with the carrier comprising carrier arms and windows;
- Fig. 4: in a schematic perspective view, a scanning pattern on the inner circumferential body surface in circles being combined with a circular feed about the longitudinal axis;
- Fig. 5: a variant of the scanning pattern according to Fig. 4 scanning parallel to the longitudinal axis combined with a circular feed about the longitudinal axis;
- Fig. 6: a further variant of the scanning pattern according to Fig. 4 and 5 scanning a random pattern combined with a circular feed about the longitudinal axis;
- Fig. 7: a further scanning pattern only in a circular way about the longitudinal axis of the treatment head.

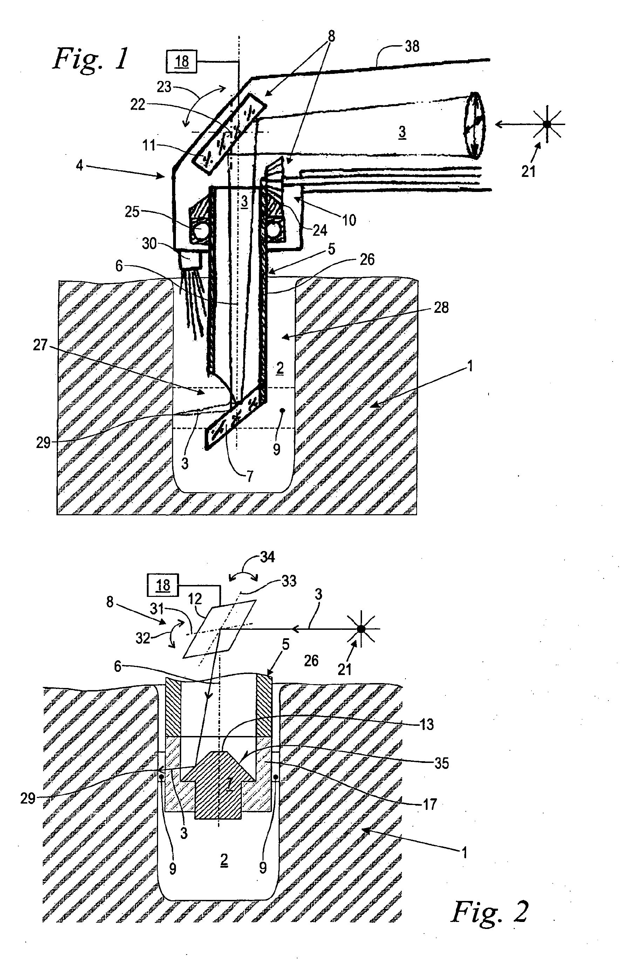

Fig. 1 shows in a schematic sectional view a first example of a laser system. The laser system comprises a handpiece 4 and a laser source 21 for generation of a laser beam 3. The laser source 21 is an Er:YAG-laser with a wavelength of 2.94 µm, but may alternatively be, depending on the treated surface of body tissue 1, an Er,Cr:YSGG-laser (2.73 µm wavelength) or a CO₂-laser (8-11 µm wave length) or a laser with any other wavelength. The laser beam 3 emitted from the laser source 21 may be guided into the hand piece 4 by means of an articulated arm or an optical fibre. It may also be expedient to place a laser source 21 in the handpiece 4.

In this embodiment, the handpiece 4 comprises a base body 38 and a treatment head 5. The treatment head 5 extends along a longitudinal axis 6. A flat deflection mirror 7 is disposed in the treatment head 5. The area of the deflection mirror 7 is disposed in a 45° angle to the longitudinal axis 6. The treatment head 5 further comprises a tube 26, to which the deflection mirror 7 is fixedly connected. The deflection mirror 7 comprises an exit side, at which an opening 27 for the emerging laser beam 3 is provided in the tube 26. The opening 27 may be covered or closed by a transparent window.

The longitudinal axis 6 of the treatment head 5 may be parallel or coaxial to the longitudinal axis of the base body 38. In this embodiment, the longitudinal axis 6 is disposed in an angle thereto, said angle being at least approximately 90° as shown. However, different angles may be chosen as well. In order to deflect the laser beam 3 after entering the hand piece 4, an additional deflection mirror is disposed in the base body 38. Said additional deflection mirror introduces the laser beam 3 into the treatment head 5 at least approximately parallel to the longitudinal axis 6. In addition, said additional deflection mirror forms a scanner 11 having a control device 18. Under the control of the control device 18, the mirror of the scanner 11 is rotationally movable about a rotational axis 22 according to an arrow 23. The rotational axis 22 is perpendicular to the axis of the incoming laser beam 3 and to the axis of the laser beam portion being reflected by the scanner 11.

As shown in Fig. 1, the laser beam 3 entering the hand piece 4 has a certain diameter, from which the laser beam 3 is focussed along its path thorough the hand piece 4 onto an impingement point 29. By means of a suitable focussing optical arrangement, the laser beam 3 may have different diameters at the impingement point 29. By adaption of said diameter and power of the laser source 29, the fluence in the impingement point 29 may be set to a desired value, thereby achieving the desired treatment result.

The laser system comprises movable deflection means 8 for the laser beam 3. In this example, wherein the additional deflection mirror is embodied as a scanner 11, said upper scanner 11 is part of the movable deflection means 8. The movable deflection means 8 additionally comprises a rotation device 10 for rotating the lower deflection mirror 7 about the longitudinal axis 6 of the treatment head 5. It may be expedient to rotate the deflection mirror 7 alone, while the further parts of the treatment head 5 remain stationary. In the shown embodiment, the entire treatment head 5 including the deflection mirror 7 is rotatably mounted at the base body 38 by means of a bearing 25, thereby being rotatable about the longitudinal axis 6. For rotatably driving the treatment head, the rotation device 10 is provided with an angular gear 24 embodied as a miter gear. By driving the rotation device 10 e.g. by means of an electric motor, the deflection mirror 7 and the entire treatment head 5 is rotated about the longitudinal axis 6. The control device 18 is additionally adapted and provided for controlling the rotation device 10 and for providing a controlled coordination between the scanner 11 and the rotation device 10.

The laser system is provided for treating body tissue 1 at an inner circumferential tissue surface 2. As an example for the body tissue 1, bone material is shown, wherein a hole 28 was mechanically drilled beforehand. The hole 28 comprises an inner body tissue surface 2 circumferentially extending about the longitudinal axis of the hole 28. In operation, the treatment head 5 is inserted in the hole 28 axially parallel to its axis; as a consequence, the longitudinal axis 6 of the treatment head 5 is disposed at least approximately parallel to the inner circumferential tissue surface 2. The laser beam 3 being reflected from the scanner 11 enters the treatment head 5 at least approximately in the direction of the longitudinal axis 6. Afterwards, the laser beam 3 is deflected radially outwards by the deflection mirror 7, which is disposed in said 45° angle referred to the longitudinal axis 6. After being reflected at the deflection mirror 7, the laser beam 3 radially emerges from the treatment head 5 in an outward direction and impinges on the inner circumferential body surface 2 on the impingement point 29.

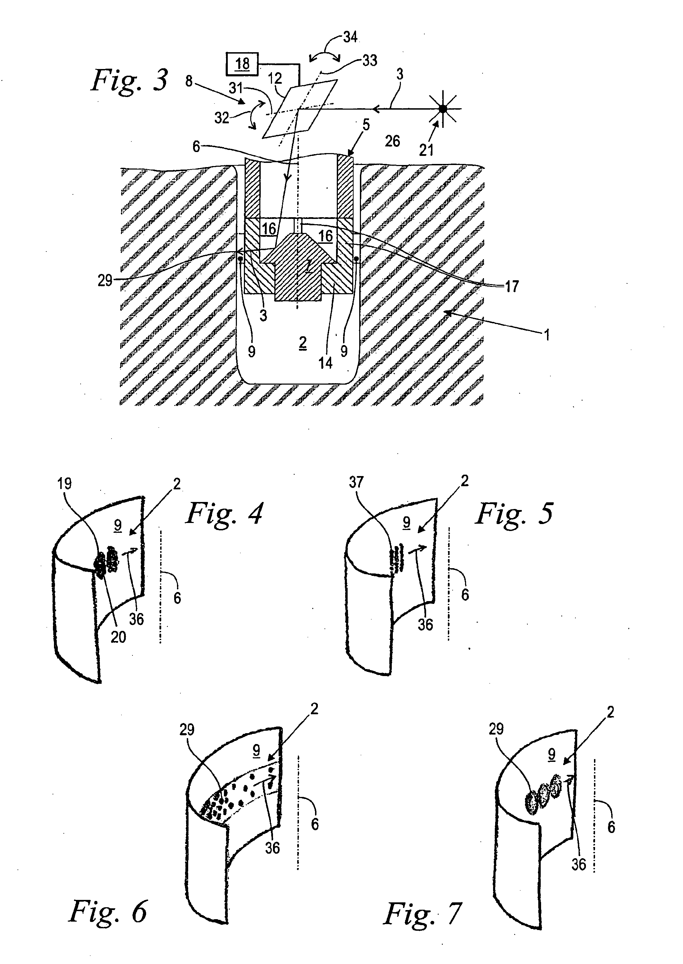

By rotating the deflection mirror 7, respectively the entire treatment head 5 the impingement point 29 is moved within the treatment area 9 of the inner circumferential tissue surface 2 in the circumferential direction both about the longitudinal axis 6 of the treatment head 5 and about the longitudinal axis of the hole 28. Hereby the treatment area 9 is scanned by the laser beam 3 in the circumferential direction. As a consequence of an additional movement of the scanner 11 about its rotational axis 22 according to the arrow 23, the treatment area 9 is additionally scanned by the laser beam 3 parallel to the longitudinal axis 6 of the treatment head 5 and to the longitudinal axis of the hole 28, respectively. By combining scanning movements both in axial and in circumferential direction, different desired scanning patterns can be achieved, as exemplarily shown in and described below with reference to Figs. 4 to 7.

For cooling and rinsing the treatment area 9, a spray device 30 is provided at the hand piece 4, by means of which air and/or water or other suitable media may be supplied to the treatment area 9, if desired.

Fig. 2 shows in a schematic sectional view a variant of the arrangement according to Fig. 1 as an example of the invention, wherein the base body 38 of Fig. 1 is not shown for the sake of simplicity. No rotation device 10 (Fig. 1) is provided in the embodiment of Fig. 2. Instead, the movable deflection means 8 comprises a scanner 12 in a form of a deflection mirror, which is rotationally movable about two axes 31, 33 perpendicular to each other according to arrows 32, 34. Like the scanner 11 of Fig. 1, the scanner 12 of Fig. 2 is controlled by the control device 18.

The deflection mirror 7 is embodied as a cone comprising a conical mirror surface 35 and an apex 13. The central axis of the conical deflection mirror 7 is disposed coaxially to the longitudinal axis 6 of the treatment head 5, wherein the apex 13 is facing the scanner 12. The aperture angle of the conical mirror surface 35 is at least approximately 90°, but may have a different value. The conical deflection mirror 7 is embodied as a metal body having a reflective, polished metal mirror surface 35. In lieu of the polished mirror surface 35, a reflective coating may be provided.

As a consequence of said aperture angle, the incoming laser beam 6 is radial outwardly reflected by the conical mirror surface 35 and impinges on the treatment area 9 of the inner circumferential tissue surface 2 at the impingement point 29. By rotationally moving the scanner 12 about its two rotational axes 31, 33, any point of the conical mirror surface 31 may be scanned referred to both the circumferential and radial direction. Thereby the radially emerging laser beam 3 may reach with its impingement point 29 any location on the treatment area 9 referred to both the circumferential direction and the axial direction, the latter being predetermined by the longitudinal axis 6.

The conical deflection mirror 7 is fixedly attached to the tube 26 of the treatment head 5 by means of a transparent carrier 17. The transparent carrier 17 may be made of an optical glass and is entirely closed in the circumferential direction about the longitudinal axis 6. Hereby the transparent carrier 17 does not only act as a carrier for the deflection mirror 7, but also acts as a protective window for the inner space of the treatment head 5, in particular including the reflective conical mirror surface 35. In addition, the laser beam 3 can unobstructedly transit the transparent carrier 17 radially outwards to the treatment area 9.

Fig. 3 shows a variant of the arrangement according to Fig. 2, wherein, as a replacement of the transparent carrier 17 (Fig. 2), a carrier 14 is provided for carrying and fixing the deflection mirror 7 to the tube 26. The carrier 14 comprises carrier arms 15 for fixing the arrangement to the tube 26, wherein the carrier arms 15 are disposed parallel to the longitudinal axis 6. Windows 16 are provided between the carrier arms 15, through which the reflected laser beam 3 emerges to the treatment area 9. The windows 16 are open, but may also be embodied as transparent protective windows of glass or any other suitable material being transparent for the laser beam 3, and having a function being comparable to the transparent carrier 17 of Fig. 2.

With respect to all other described and/or shown features and reference signs, and if not otherwise stated, the arrangements of Fig. 2 and 3 concur with each other and with the arrangement of Fig. 1.

The inventive laser system including the control device 18 is adapted to the operated as follows: In general, the treatment area 9 of the inner circumferential body surface 2 is scanned in particular patterns. Said scanning is performed to remove residual smear layers from body tissue 1 being e. g. a hard bone material, in which holes 28 (Fig. 1 to 3) were mechanically drilled beforehand. In addition, a certain surface structure of the inner circumferential tissue surface and/or a correction of the hole profile may be achieved. It is further possible to use the inventive arrangement and method for treatment of soft body tissue such as skin or the like in surgical cuts or existing body openings, as may be desired e. g. along with vaginal treatments. Related preferred scanning patterns are schematically depicted in Figs. 4 to 7, wherein sections of the inner circumferential tissue surface 2 are perspectively shown. As in Figs. 1 to 3, the longitudinal axis 6 of the treatment head 5 is disposed at least approximately parallel to the circumferential tissue surface 2 and its treatment area 9.

For certain treatment tasks, sufficiently high fluencies of the laser beam 3 are required. In such cases, the laser beam 3 is focussed on the impingement point 29 with a small, nearly pinpoint shaped diameter, wherein said diameter is small compared to the axial extension of the treatment area 9. This requires a scanning of the treatment area 9 both in an axial and a circumferential direction, as shown in Fig. 4 to 6.

According to the example of Fig. 4, the treatment area 9 of the inner circumferential tissue surface 2 is scanned in a pattern, in which the impingement point 29 (Figs. 1 to 3) is guided on circles 19, wherein the circles 19 including their centers 20 are disposed on the treatment area 9. The circles 19 may be scanned along their circumference or across their entire circular area. In addition to said circular scanning about the centers 20, a circular feed of the impingement point 29 about the longitudinal axis 6 according to an arrow 36 is provided; in consequence thereof, the treatment area 9 is entirely scanned both in a circumferential and an axial direction related to the longitudinal axis 6.

In the embodiment according to Fig. 5, lines 37 are provided instead of circles 19 (Fig. 4), said lines 37 being disposed parallel to the longitudinal axis 6, and along which the treatment area 9 is scanned. In addition thereto, a circular feed of the impingement point 29 about the longitudinal axis in the direction of the arrow 36 is provided.

A further embodiment is shown in Fig. 6, according to which the treatment area 9 is scanned in a random pattern. The impingement points 29 are randomly spread over the treatment area 9. Again, an additional feed in the circumferential direction about the longitudinal axis 6 according to the arrow 36 is provided.

In the embodiments according to Fig. 4 to 6, two separate scanning patterns are provided. The first scanning pattern is either circular (Fig. 4), linear (Fig. 5) or random (Fig. 6). The second scanning pattern is circular about the longitudinal axis 6. It may be expedient to switch between both types of patterns back and forth in order to scan the entire treatment area 9. As an alternative, it may be expedient to continuously superpose both scanning patterns for scanning the entire treatment area 9.

In a further variant of the invention, as schematically shown in Fig. 7, an axial scanning of the treatment area 9 may be omitted. This is in particular suitable for the case, wherein, instead of nearly pinpoint shaped impingement points 29 (Figs. 4 to 6), planar impingement points 29 of larger diameter are projected onto the treatment area 9, which results in an axial extension of the scanned treatment area 9 without axial scanning movement components. Hereby the treatment area 9 is solely scanned in a circular manner about the longitudinal axis 6, according to the arrow 36. In the embodiments according to Figs. 2 and 3, this may be achieved by a suitable control of the scanner 12 and by a circular scanning of the conical deflection mirror 7. In the embodiment according to Fig. 1, said circular scanning pattern is achieved by the rotational movement of the treatment head 5 without any further measures. Only the rotation device 10 and the rotational movement of deflection mirror 7 is required as a movable deflection means 8, while the scanner 11 may be omitted.

## Claims

1. Laser system for treatment of body tissue (21) on an inner circumferential tissue surface (2), comprising
a laser source (1) for the generation of a laser beam (3) and a handpiece (4) with a treatment head (5),
wherein the treatment head (5) extends along a longitudinal axis (6), wherein during operation the laser beam (3) enters the treatment head (5) in the direction of the longitudinal axis (6), wherein a deflection mirror (7) is disposed in the treatment head (5) and guides the laser beam (3) radially outwards out of the treatment head (5),
wherein a movable deflection means (8) for the laser beam (3) is provided,
wherein the movable deflection means (8) comprise a scanner (12) being movable about two axes, wherein the deflection mirror (7) has a conical shape and is disposed with its apex (13) facing the scanner (12), and wherein the scanner (12) is controlled to provide a circular scanning in cooperation with the conical deflection mirror (7).

2. Laser system according to claim 1,
**characterized in that** the laser system comprises a carrier (14), and **in that** the deflection mirror (7) is fixed to the treatment head (5) by means of the carrier (14), wherein the carrier (14) comprises carrier arms (15) and windows (16) between the carrier arms (15) for the emerging laser beam (3).

3. Laser system according to claim 1,
**characterized in that** the laser system comprises a carrier (17), and **in that** the deflection mirror (7) is fixed to the treatment head (5) by means of the carrier (17), wherein the carrier (17) is transparent and closed in the circumferential direction.

4. Laser system according to one of claim 1 to 3,
**characterized in that** the deflection means (8) comprises a control device (18) adapted to scan the treatment area (9) by the laser beam (3) on circles (19) with centres (20) on the treatment area (9), wherein the laser beam (3) is subjected to a circular feed about the longitudinal axis of the treatment head (5).

5. Laser system according to one of claim 1 to 3,
**characterized in that** the deflection means (8) comprises a control device (18) adapted to scan the treatment area (9) by the laser beam (3) parallel to the longitudinal axis of the treatment head (5), wherein the laser beam (3) is subjected to a circular feed about the longitudinal axis of the treatment head (5).

6. Laser system according to one of claim 1 to 3,
**characterized in that** the deflection means (8) comprises a control device (18), adapted to scan the treatment area (9) by the laser beam (3) in a random pattern, wherein the laser beam (3) is subjected to a circular feed about the longitudinal axis of the treatment head (5).

7. Laser system according to one of claim 1 to 3,
**characterized in that** the deflection means (8) comprises a control device (18), adapted to scan the treatment area (9) by the laser beam (3) solely in a circular way about the longitudinal axis of the treatment head (5).

## Patentansprüche

1. Lasersystem für die Behandlung von Körpergewebe (21) auf einer inneren, umlaufenden Gewebeoberfläche (2), umfassend eine Laserquelle (1) für die Erzeugung eines Laserstrahls (3) und ein Handstück (4) mit einem Behandlungskopf (5), wobei sich der Behandlungskopf (5) entlang einer Längsachse (6) erstreckt, wobei während des Betriebs der Laserstrahl (3) in den Behandlungskopf (5) in Richtung der Längsachse (6) eintritt, wobei ein Umlenkspiegel (7) im Behandlungskopf (5) angeordnet ist und den Laserstrahl (3) radial auswärts aus dem Behandlungskopf (5) herausführt, und wobei bewegliche Umlenkmittel (8) für den Laserstrahl (3) vorgesehen sind,
**dadurch gekennzeichnet, dass** die beweglichen Umlenkmittel (8) einen um zwei Achsen beweglichen Scanner (12) aufweisen, wobei der Umlenkspiegel (7) eine konische Form hat und mit seiner Spitze dem Scanner (12) zugewandt angeordnet ist, und wobei der Scanner (12) für zumindest ein kreisförmiges Scannen des konischen Umlenkspiegels gesteuert ist.

2. Lasersystem nach Anspruch 1,
**dadurch gekennzeichnet, dass** das Lasersystem einen Träger (14) aufweist, und dass der Umlenkspiegel (7) mittels des Trägers (14) am Behandlungskopf (5) befestigt ist, wobei der Träger (14) Trägerarme (15) und zwischen den Trägerarmen (15) Fenster für den austretenden Laserstrahl (3) aufweist.

3. Lasersystem nach Anspruch 1,
**dadurch gekennzeichnet, dass** das Lasersystem einen Träger (14) aufweist, und dass der Umlenkspiegel (7) mittels des Trägers (14) am Behandlungskopf (5) befestigt ist, wobei der Träger (14) transparent und in der Umfangsrichtung geschlossen ist.

4. Lasersystem nach einem der Ansprüche 1 bis 3,
**dadurch gekennzeichnet, dass** die Umlenkmittel (8) ein Steuergerät (18) aufweisen, wobei das Steuergerät (8) in einer solchen Weise angepasst ist, dass die Behandlungsfläche (9) durch den Laserstrahl (3) auf Kreisen (19) mit Mittelpunkten (20) auf der Behandlungsfläche (9) gescannt wird, wobei der Laserstrahl (3) einem kreisförmigen Vorschub um die Längsachse des Behandlungskopfes (5) ausgesetzt ist.

5. Lasersystem nach einem der Ansprüche 1 bis 3,
**dadurch gekennzeichnet, dass** die Umlenkmittel (8) ein Steuergerät (18) aufweisen, wobei das Steuergerät (8) in einer solchen Weise angepasst ist, dass die Behandlungsfläche (9) durch den Laserstrahl (3) parallel zur Längsachse des Behandlungskopfes (5) gescannt wird, wobei der Laserstrahl (3) einem kreisförmigen Vorschub um die Längsachse des Behandlungskopfes (5) ausgesetzt ist.

6. Lasersystem nach einem der Ansprüche 1 bis 3,
**dadurch gekennzeichnet, dass** die Umlenkmittel (8) ein Steuergerät (18) aufweisen, wobei das Steuergerät (8) in einer solchen Weise angepasst ist, dass die Behandlungsfläche (9) durch den Laserstrahl (3) in einem beliebigen Muster gescannt wird, wobei der Laserstrahl (3) einem kreisförmigen Vorschub um die Längsachse des Behandlungskopfes (5) ausgesetzt ist.

7. Lasersystem nach einem der Ansprüche 1 bis 3,
**dadurch gekennzeichnet, dass** die Umlenkmittel (8) ein Steuergerät (18) aufweisen, wobei das Steuergerät (8) in einer solchen Weise angepasst ist, dass die Behandlungsfläche (9) durch den Laserstrahl (3) ausschließlich auf einem kreisförmigen Weg um die Längsachse des Behandlungskopfes (5) gescannt wird.

## Revendications

1. Système laser pour le traitement de tissu corporel (1) sur une surface de tissu circonférentielle intérieure (2), comprenant une source laser (21) pour produire un faisceau laser (3), et une pièce à main (4) avec une tête de traitement (5), étant précisé que la tête de traitement (5) s'étend le long d'un axe longitudinal (6), que pendant l'opération, le faisceau laser (3) entre dans la tête de traitement (5) dans le sens de l'axe longitudinal (6), qu'un miroir déflecteur (7) est disposé dans la tête de traitement (5) et guide le faisceau laser (3) radialement vers l'extérieur de la tête de traitement (5), qu'il est prévu des moyens déflecteurs mobiles (8) pour le faisceau laser (3), que les moyens déflecteurs mobiles (8) comprennent un scanner (12) mobile sur deux axes, que le miroir déflecteur (7) présente une forme conique et est disposé avec son sommet (13) face au scanner (12), et que le scanner (12) est commandé pour fournir un balayage circulaire en coopération avec le miroir déflecteur conique (7).

2. Système laser selon la revendication 1,
**caractérisé en ce que** le système laser comprend un support (14), et **en ce que** le miroir déflecteur (7) est fixé à la tête de traitement (5) à l'aide du support (14), étant précisé que le support (14) comprend des bras de support (15) et des fenêtres (16), entre les bras de support (15), pour le faisceau laser qui sort (3).

3. Système laser selon la revendication 1,
**caractérisé en ce que** le système laser comprend un support (17), et **en ce que** le miroir déflecteur (7) est fixé à la tête de traitement (5) à l'aide du support (17), étant précisé que le support (17) est transparent et fermé dans le sens circonférentiel.

4. Système laser selon l'une des revendications 1 à 3,
**caractérisé en ce que** les moyens déflecteurs (8) comprennent un dispositif de commande (18) pour balayer la zone de traitement (9) à l'aide du faisceau laser (3) sur des cercles (19) avec des centres (20) sur la zone de traitement (9), étant précisé que le faisceau laser (3) est soumis à une amenée circulaire autour de l'axe longitudinal de la tête de traitement (5).

5. Système laser selon l'une des revendications 1 à 3,
**caractérisé en ce que** les moyens déflecteurs (8) comprennent un dispositif de commande (18) pour balayer la zone de traitement (9) à l'aide du faisceau laser (3) parallèlement à l'axe longitudinal de la tête de traitement (5), étant précisé que le faisceau laser (3) est soumis à une amenée circulaire autour de l'axe longitudinal de la tête de traitement (5).

6. Système laser selon l'une des revendications 1 à 3,
**caractérisé en ce que** les moyens déflecteurs (8) comprennent un dispositif de commande (18) apte à balayer la zone de traitement (9) à l'aide du faisceau laser (3) suivant un modèle aléatoire, étant précisé que le faisceau laser (3) est soumis à une amenée circulaire autour de sur l'axe longitudinal de la tête de traitement (5).

7. Système laser selon l'une des revendications 1 à 3,
**caractérisé en ce que** les moyens déflecteurs (8) comprennent un dispositif de commande (18) apte à balayer la zone de traitement (9) à l'aide du faisceau laser (3) uniquement d'une manière circulaire autour de l'axe longitudinal de la tête de traitement (5).
